# EUROPEAN PATENT SPECIFICATION

(11) **EP 4 265 717 B1**
(45) Date of publication and mention of the grant of the patent: **05.11.2025**
(21) Application number: 22871086.9
(22) Date of filing: 23.12.2022
(51) Int. Cl.: C12N 5/20, C07K 16/28, C12N 15/13, G01N 33/577, G01N 33/68, C07K 16/18, G01N 33/569

(54) **ANTI-MOBILIZED COLISTIN RESISTANCE (MCR)-1/MCR-2 PROTEIN MOUSE HYBRIDOMA CELL LINE, MONOCLONAL ANTIBODY (mAb) AND APPLICATION**
ANTI-MOBILISIERTER COLISTIN-RESISTENZ (MCR)-1/MCR-2 PROTEIN HYBRIDOMAZELLINIE DER MAUS, MONOKLONALER ANTIKÖRPER (mAB) UND ANWENDUNG
SOUCHE CELLULAIRE D'HYBRIDOME PROTÉIQUE ANTI-MCR-1/MCR-2 DE SOURIS, ANTICORPS MONOCLONAL ET APPLICATION

(30) Priority: 01.03.2022 CN 202210189230
(43) Date of publication of application: 25.10.2023
(73) Proprietor: Tianjin Era Biology Technology Co., Ltd., Tianjin 300457 (CN); BEIJING GOLD MOUNTAINRIVER TECH DEVELOPMENT CO., LTD., Beijing 100081 (CN); Genobio Pharmaceutical Co., Ltd., Binhai New Area Tianjin 300480 (CN)
(72) Inventor: LI, Keke, Tianjin 300480 (CN); YUAN, Qinghua, Tianjin 300480 (CN); HE, Yongsheng, Tianjin 300480 (CN); CHEN, Xiaoling, Tianjin 300480 (CN); XIA, Bin, Tianjin 300480 (CN); WANG, Yamiao, Tianjin 300480 (CN); KONG, Di, Tianjin 300480 (CN); WANG, Siyi, Tianjin 300480 (CN); YANG, Yanlin, Tianjin 300480 (CN)
(74) Representative: Bayramoglu et al.
(86) International application number: PCT/CN2022/141244
(87) International publication number: WO 2023/165230

(56) References cited:
- CN-A- 111 487 417
- CN-A- 113 416 710
- CN-A- 114 250 203
- CN-A- 114 317 455
- US-B2- 11 161 896
- WANG JIAYI ET AL: "Conclusions", vol. 10, no. 1, 17 October 2019 (2019-10-17), XP055799019, Retrieved from the Internet <URL:https://jasbsci.biomedcentral.com/track/pdf/10.1186/s40104-019-0389-7.pdf> DOI: 10.1186/s40104-019-0389-7
- DATABASE PROTEIN ANONYMOUS : "Chain B, Fab", XP093087164, retrieved from NCBI
- LI HUI, WANG YINGYU, CHEN QIYAN, XIA XI, SHEN JIANZHONG, WANG YANG, SHAO BING: "Identification of Functional Interactome of Colistin Resistance Protein MCR-1 in Escherichia coli", FRONTIERS IN MICROBIOLOGY, vol. 11, XP093087165, DOI: 10.3389/fmicb.2020.583185
- LIU, YANHUA ET AL.: "Research Progress of Plasmid-mediated Colistin Resistance Gene Mcr", EXPERIMENTAL AND LABORATORY MEDICINE, CN, vol. 37, no. 3, 30 June 2019 (2019-06-30), CN , pages 347 - 351, XP009548780, ISSN: 1674-1129, DOI: 10.3969/j.issn.1674-1129.2019.03.001

## Description

### TECHNICAL FIELD

The present invention belongs to the technical field of antibody preparation, in particular to an anti-mobilized colistin resistance (MCR)-1/MCR-2 protein mouse hybridoma cell line, a monoclonal antibody (mAb), and an application.

### BACKGROUND

Currently, polymyxin is the last choice for the clinical treatment of multi-drug-resistant and pan-drug-resistant Gram-negative bacterial infections. In recent years, due to the increasing clinical use of polymyxin, the number of drug-resistant strains is also increasing year by year. Although the resistance of bacteria to polymyxin is not strong at present, it may lead to the situation of no medicine being available. How to effectively curb the spread of drug resistance has become a global public health issue. In multi-drug resistance mechanisms, the MCR gene that mediates the addition of phosphoethanolamine (PEtn) in lipid A was found to be in multiple plasmids and could be horizontally transmitted in different bacteria through the plasmids, or even co-exist in the same plasmid with other drug resistance genes. Resulting in multi-drug resistance mechanisms after expression. In addition, the unreasonable application of antibiotics makes the resistance of bacteria continuously enhanced, which causes great trouble for clinicians to choose antibiotics.

The detection of MCR-mediated polymyxin resistance is mainly through molecular biological methods in laboratories. The development of MCR protein rapid diagnostic products with independent intellectual property rights is of great significance for the rapid identification of polymyxin-resistant strains and their resistance mechanisms, the optimization of polymyxin treatment regimens, and the extension of the service life of polymyxin as the last-line of treatment drugs. US11161896B2 discloses antibodies that are capable of binding both MCR-1 and MCR-2 proteins.

### SUMMARY

In order to solve the above technical problems, the present invention provides an anti-MCR-1/MCR-2 protein mouse hybridoma cell line, a mAb, and an application.

The technical solution adopted by the present invention is as follows: an anti-MCR-1/MCR-2 protein mouse hybridoma cell line named 2DG5 with the accession number of CGMCC No. 23034; or an anti-MCR-1/MCR-2 protein mouse hybridoma cell line named 1IB10 with the accession number of CGMCC No. 23032.

An anti-MCR-1/MCR-2 protein mouse mAb 2DG5 includes a light chain variable region and a heavy chain variable region. The light chain variable region includes CDRL1 as shown in SEQ ID NO: 1, CDRL2 as shown in SEQ ID NO: 2, and CDRL3 as shown in SEQ ID NO: 3. The heavy chain variable region includes CDRH1 as shown in SEQ ID NO: 4, CDRH2 as shown in SEQ ID NO: 5, and CDRH3 as shown in SEQ ID NO: 6;
SEQ ID NO: 1 RASQDINNYLN (CDRL1)
SEQ ID NO: 2 YTSRLHS (CDRL2)
SEQ ID NO: 3 QQGKTIHPWT (CDRL3)
SEQ ID NO: 4 GYSITSDYAWN (CDRH1)
SEQ ID NO: 5 QISDSAVTTYNPSLKS (CDRH2)
SEQ ID NO: 6 RNFYGYNFFDY (CDRH3)
   or,
an anti-MCR-1/MCR-2 protein mouse mAb 1IB10 is provided, the light chain variable region of which includes CDRL1 as shown in SEQ ID NO: 11, CDRL2 as shown in SEQ ID NO: 12, and CDRL3 as shown in SEQ ID NO: 13, and the heavy chain variable region of which includes CDRH1 as shown in SEQ ID NO: 14, CDRH2 as shown in SEQ ID NO: 15, and CDRH3 as shown in SEQ ID NO: 16.
SEQ ID NO: 11 RSSQNIVCSYGNPCLE (CDRL1)
SEQ ID NO: 12 KVSNRFS (CDRL2)
SEQ ID NO: 13 FQSSHVPWT (CDRL3)
SEQ ID NO: 14 GYTFTSYVMH (CDRH1)
SEQ ID NO: 15 YFNPYNDGANYNEKFKG (CDRH2)
SEQ ID NO: 16 GPYGNYAMDY (CDRH3)

Preferably, an amino acid sequence of the light chain variable region of the antibody 2DG5 is shown in SEQ ID NO: 7, and an amino acid sequence of the heavy chain variable region of the antibody 2DG5 is shown in SEQ ID NO: 9;
SEQ ID NO: 7 
DIQMTQTTSSLSASLGDRVTINCRASQDINNYLNWYQQKPDGTVKLLIYYTSRL
HSGVPSRFSGSGSRTDYSLTISNLEREDIATYFCQQGKTIHPWTFGGGTKLDIKRA
SEQ ID NO: 9 
VKLQESGPGLVKPSQSLSLTCTVTGYSITSDYAWNWIRQFPGNKLEWMGQISDS
AVTTYNPSLKSRISINRDTSKNQFFLQLNSVTPEDTATYYCARRNFYGYNFFDYWGR
GTSLTVSSAK
or,
an amino acid sequence of the light chain variable region of the antibody 1IB10 is shown in SEQ ID NO: 17, and an amino acid sequence of the heavy chain variable region of the antibody 1IB10 is shown in SEQ ID NO: 19;
SEQ ID NO: 17 
DVLMTQTPLSLPVSLGDQASISCRSSQNIVCSYGNPCLEWYLQKPGQSPKLLIYK
VSNRFSGVPDRFSGSGSGTDFTLKISRVEAEDLGVYYCFQSSHVPWTFGGGTELEIKR
SEQ ID NO: 19

VKLQQSGPELVKPGASVKMSCRASGYTFTSYVMHWVKQKPGQGLEWIGYFNP
YNDGANYNEKFKGKATLSSDKSSGTAYMDLSSLTSEDSAVYYCARGPYGNYAMDY
WGQGTSVTVSSA

Preferably, the antibody 2DG5 is produced by the anti-MCR-1/MCR-2 protein mouse hybridoma cell line with the accession number of CGMCC No. 23034;
or,
the antibody 1IB10 is produced by the anti-MCR-1/MCR-2 protein mouse hybridoma cell line with the accession number of CGMCC No. 23032.

A nucleic acid molecule includes a nucleotide that encodes the anti-MCR-1/MCR-2 protein mouse mAb.

Preferably, a nucleotide sequence of the nucleic acid molecule that encodes the light chain variable region of the antibody 2DG5 is shown in SEQ ID NO: 8, and a nucleotide sequence of the nucleic acid molecule that encodes the heavy chain variable region of the antibody 2DG5 is shown in SEQ ID NO: 10;
SEQ ID NO: 8 
GATATCCAGATGACACAGACTACATCCTCCCTGTCTGCCTCTCTGGGAGACA
GAGTCACCATCAATTGCAGGGCAAGTCAGGACATTAACAATTATTTAAACTGGT
ATCAGCAGAAACCAGATGGAACTGTTAAACTCCTGATCTACTACACTTCAAGATT
ACATTCAGGAGTCCCATCAAGGTTCAGCGGCAGTGGGTCTAGAACAGATTATTCT
CTCACCATTAGCAACCTGGAACGAGAAGATATTGCCACTTACTTTTGCCAACAGG
GTAAAACGATTCATCCGTGGACGTTCGGTGGAGGCACCAAGCTGGACATCAAAC
GGGCT
SEQ ID NO: 10 
GTGAAGCTGCAGGAGTCAGGACCTGGCCTGGTGAAACCTTCTCAGTCTCTGT
CCCTCACCTGCACTGTCACTGGCTACTCAATCACCAGTGATTATGCCTGGAACTG
GATCCGGCAGTTTCCAGGAAACAAACTGGAGTGGATGGGCCAGATAAGCGACAG
TGCTGTCACTACCTACAACCCATCTCTCAAAAGTCGAATCTCTATCAATCGAGAC
ACATCCAAGAACCAGTTCTTCCTGCAGTTGAATTCTGTGACTCCTGAGGACACAG
CCACATATTACTGTGCAAGAAGGAACTTCTACGGGTACAATTTTTTTGACTACTG
GGGCCGAGGCACCTCTCTCACTGTCTCTTCAGCCAAA
or,
a nucleotide sequence of the nucleic acid molecule that encodes the light chain variable region of the antibody 1IB10 is shown in SEQ ID NO: 18, and a nucleotide sequence of the nucleic acid molecule that encodes the heavy chain variable region of the antibody 1IB10 is shown in SEQ ID NO: 20.
SEQ ID NO: 20 
GATGTTTTGATGACCCAGACGCCACTCTCCCTGCCTGTCAGTCTTGGAGATC
AAGCCTCCATCTCTTGCAGATCTAGTCAGAACATTGTATGTAGTTATGGAAACCC
CTGTTTAGAATGGTACCTGCAGAAACCAGGCCAGTCTCCAAAGCTCCTGATCTAC
AAAGTTTCCAACCGATTTTCTGGGGTCCCAGACAGGTTCAGTGGCAGTGGATCAG
GGACAGATTTCACACTCAAGATCAGCAGAGTGGAGGCTGAGGATCTGGGAGTTT
ATTACTGCTTTCAAAGTTCACATGTTCCGTGGACGTTCGGTGGAGGCACCGAGCT
GGAAATCAAACGG
SEQ ID NO: 20

GTGAAGCTGCAGCAGTCTGGACCTGAGCTGGTAAAGCCTGGGGCTTCAGTG

AAGATGTCCTGCAGGGCTTCTGGATACACATTCACTAGCTATGTTATGCACTGGG
TGAAGCAGAAGCCTGGGCAGGGCCTTGAGTGGATTGGATATTTTAATCCTTACAA
TGACGGTGCTAACTACAATGAGAAGTTCAAAGGCAAGGCCACACTGTCTTCAGA
CAAATCCTCCGGCACAGCCTATATGGACCTCAGCAGCCTGACCTCTGAGGACTCT
GCGGTCTATTATTGTGCGAGAGGCCCCTATGGTAACTATGCTATGGACTACTGGG
GTCAAGGAACCTCAGTCACCGTCTCCTCAGCC

An application of the anti-MCR-1/MCR-2 protein mouse mAb in the preparation of reagents for detection of an MCR-1 or MCR-2 protein.

Preferably, the anti-MCR-1/MCR-2 protein mouse mAb is used to prepare an *in vitro* diagnostic kit or a microfluidic chip. The *in vitro* diagnostic kit is a colloidal gold immunoassay kit, a chemiluminescence kit, a radioimmunoassay kit, an enzyme-linked immunoassay kit, or a fluorescence immunoassay kit.

Preferably, a double-antibody sandwich immuno-colloidal gold test card is prepared, where the antibody 2DG5 is used as a coated antibody, and the antibody 1IB10 is used as a gold-labeled antibody;
or, the antibody 1IB10 is used as the coated antibody, and the antibody 2DG5 is used as the gold-labeled antibody.

The advantages and positive effects of the present invention are as follows: the present invention provides two anti-MCR-1/MCR-2 protein mouse hybridoma cell lines, which can respectively produce two anti-MCR-1/MCR-2 protein mouse mAbs. After systematic evaluation, including evaluations on the antibody subtype and titer, kit sensitivity, specificity, and stability, it is found that the anti-MCR-1/MCR-2 protein mouse mAbs have good performance in all aspects and the titer reaches more than 1:1280000, thereby the anti-anti-MCR-1/MCR-2 protein mouse mAbs are suitable for use as an immunodiagnostic reagent in the preparation of an *in vitro* diagnostic kit for the detection of polymyxin-resistant strains.

### BRIEF DESCRIPTION OF THE DRAWINGS

FIG. 1 is an image showing the protein electrophoresis of MCR-1/MCR-2 proteins;
FIG. 2 is an image showing the protein electrophoresis of anti-MCR-1/MCR-2 protein mouse mAbs;
FIG. 3 shows the detection results of the MCR-1/MCR-2 proteins test card by a colloidal gold method.

Biological material: 2DG5 was deposited in China General Microbiological Culture Collection Center (CGMCC) on September 24, 2021, with the accession number of CGMCC No. 23034;
Biological material: 1IB10 was deposited in CGMCC on September 24, 2021, with the accession number of CGMCC No. 23032.

### DETAILED DESCRIPTION OF THE EMBODIMENTS

The embodiments of the present invention are explained below.

The present invention relates to an anti-MCR-1/MCR-2 protein mouse hybridoma cell line, the biological material of which is named 2DG5 and belongs to the hybridoma cell. 2DG5 was deposited in CGMCC on September 24, 2021, with the accession number of CGMCC No. 23034 and is tested to be alive. Another anti-MCR-1/MCR-2 protein mouse hybridoma cell line is further provided, the biological material of which is named 1IB10 and belongs to the hybridoma cell. 1IB10 was deposited in CGMCC on September 24, 2021, with the accession number of CGMCC No. 23032 and is tested to be alive.

The antibody 2DG5 produced by the anti-MCR-1/MCR-2 protein mouse hybridoma cell line includes a light chain variable region and a heavy chain variable region. The light chain variable region includes CDRL1 as shown in SEQ ID NO: 1, CDRL2 as shown in SEQ ID NO: 2, and CDRL3 as shown in SEQ ID NO: 3. The heavy chain variable region includes CDRH1 as shown in SEQ ID NO: 4, CDRH2 as shown in SEQ ID NO: 5, and CDRH3 as shown in SEQ ID NO: 6.
SEQ ID NO: 1 RASQDINNYLN (CDRL1)
SEQ ID NO: 2 YTSRLHS (CDRL2)
SEQ ID NO: 3 QQGKTIHPWT (CDRL3)
SEQ ID NO: 4 GYSITSDYAWN (CDRH1)
SEQ ID NO: 5 QISDSAVTTYNPSLKS (CDRH2)
SEQ ID NO: 6 RNFYGYNFFDY (CDRH3)

An amino acid sequence of the light chain variable region of the antibody 2DG5 is shown in SEQ ID NO: 7, and an amino acid sequence of the heavy chain variable region of the antibody 2DG5 is shown in SEQ ID NO: 9.
SEQ ID NO: 7 
DIQMTQTTSSLSASLGDRVTINCRASQDINNYLNWYQQKPDGTVKLLIYYTSRL
HSGVPSRFSGSGSRTDYSLTISNLEREDIATYFCQQGKTIHPWTFGGGTKLDIKRA
SEQ ID NO: 9

VKLQESGPGLVKPSQSLSLTCTVTGYSITSDYAWNWIRQFPGNKLEWMGQISDS
AVTTYNPSLKSRISINRDTSKNQFFLQLNSVTPEDTATYYCARRNFYGYNFFDYWGR
GTSLTVSSAK

A nucleotide sequence encoding the light chain variable region of the antibody 2DG5 is shown in SEQ ID NO: 8, and a nucleotide sequence encoding the heavy chain variable region of the antibody 2DG5 is shown in SEQ ID NO: 10.
SEQ ID NO: 8 
GATATCCAGATGACACAGACTACATCCTCCCTGTCTGCCTCTCTGGGAGACA
GAGTCACCATCAATTGCAGGGCAAGTCAGGACATTAACAATTATTTAAACTGGT
ATCAGCAGAAACCAGATGGAACTGTTAAACTCCTGATCTACTACACTTCAAGATT
ACATTCAGGAGTCCCATCAAGGTTCAGCGGCAGTGGGTCTAGAACAGATTATTCT
CTCACCATTAGCAACCTGGAACGAGAAGATATTGCCACTTACTTTTGCCAACAGG
GTAAAACGATTCATCCGTGGACGTTCGGTGGAGGCACCAAGCTGGACATCAAAC
GGGCT
SEQ ID NO: 10

GTGAAGCTGCAGGAGTCAGGACCTGGCCTGGTGAAACCTTCTCAGTCTCTGT
CCCTCACCTGCACTGTCACTGGCTACTCAATCACCAGTGATTATGCCTGGAACTG
GATCCGGCAGTTTCCAGGAAACAAACTGGAGTGGATGGGCCAGATAAGCGACAG
TGCTGTCACTACCTACAACCCATCTCTCAAAAGTCGAATCTCTATCAATCGAGAC
ACATCCAAGAACCAGTTCTTCCTGCAGTTGAATTCTGTGACTCCTGAGGACACAG
CCACATATTACTGTGCAAGAAGGAACTTCTACGGGTACAATTTTTTTGACTACTG
GGGCCGAGGCACCTCTCTCACTGTCTCTTCAGCCAAA

The antibody 1IB10 produced by the anti-MCR-1/MCR-2 protein mouse hybridoma cell line is provided, the light chain variable region of which includes CDRL1 as shown in SEQ ID NO: 11, CDRL2 as shown in SEQ ID NO: 12, and CDRL3 as shown in SEQ ID NO: 13, the heavy chain variable region of which includes CDRH1 as shown in SEQ ID NO: 14, CDRH2 as shown in SEQ ID NO: 15, and CDRH3 as shown in SEQ ID NO: 16.
SEQ ID NO: 11 RSSQNIVCSYGNPCLE (CDRL1)
SEQ ID NO: 12 KVSNRFS (CDRL2)
SEQ ID NO: 13 FQSSHVPWT (CDRL3)
SEQ ID NO: 14 GYTFTSYVMH (CDRH1)
SEQ ID NO: 15 YFNPYNDGANYNEKFKG (CDRH2)
SEQ ID NO: 16 GPYGNYAMDY (CDRH3)

An amino acid sequence of the light chain variable region of the antibody 1IB10 is shown in SEQ ID NO: 17, and an amino acid sequence of the heavy chain variable region of the antibody 1IB10 is shown in SEQ ID NO: 19;
SEQ ID NO: 17 
DVLMTQTPLSLPVSLGDQASISCRSSQNIVCSYGNPCLEWYLQKPGQSPKLLIYK
VSNRFSGVPDRFSGSGSGTDFTLKISRVEAEDLGVYYCFQSSHVPWTFGGGTELEIKR
SEQ ID NO: 19

VKLQQSGPELVKPGASVKMSCRASGYTFTSYVMHWVKQKPGQGLEWIGYFNP
YNDGANYNEKFKGKATLSSDKSSGTAYMDLSSLTSEDSAVYYCARGPYGNYAMDY
WGQGTSVTVSSA

A nucleotide sequence encoding the light chain variable region of the antibody 1IB10 is shown in SEQ ID NO: 18, and a nucleotide sequence encoding the heavy chain variable region of the antibody 1IB10 is shown in SEQ ID NO: 20.
SEQ ID NO: 18 
GATGTTTTGATGACCCAGACGCCACTCTCCCTGCCTGTCAGTCTTGGAGATC
AAGCCTCCATCTCTTGCAGATCTAGTCAGAACATTGTATGTAGTTATGGAAACCC
CTGTTTAGAATGGTACCTGCAGAAACCAGGCCAGTCTCCAAAGCTCCTGATCTAC
AAAGTTTCCAACCGATTTTCTGGGGTCCCAGACAGGTTCAGTGGCAGTGGATCAG
GGACAGATTTCACACTCAAGATCAGCAGAGTGGAGGCTGAGGATCTGGGAGTTT
ATTACTGCTTTCAAAGTTCACATGTTCCGTGGACGTTCGGTGGAGGCACCGAGCT
GGAAATCAAACGG
SEQ ID NO: 20

GTGAAGCTGCAGCAGTCTGGACCTGAGCTGGTAAAGCCTGGGGCTTCAGTG

AAGATGTCCTGCAGGGCTTCTGGATACACATTCACTAGCTATGTTATGCACTGGG
TGAAGCAGAAGCCTGGGCAGGGCCTTGAGTGGATTGGATATTTTAATCCTTACAA
TGACGGTGCTAACTACAATGAGAAGTTCAAAGGCAAGGCCACACTGTCTTCAGA
CAAATCCTCCGGCACAGCCTATATGGACCTCAGCAGCCTGACCTCTGAGGACTCT
GCGGTCTATTATTGTGCGAGAGGCCCCTATGGTAACTATGCTATGGACTACTGGG
GTCAAGGAACCTCAGTCACCGTCTCCTCAGCC

The systematic evaluation of the anti-MCR-1/MCR-2 protein mouse mAbs was performed, including evaluations on the antibody subtype and titer, kit sensitivity, specificity, and stability. MCR-1 protein is similar to MCR-2 protein, and the anti-MCR-1/MCR-2 protein mouse mAbs prepared by the present invention can specifically recognize the MCR-1 protein and the MCR-2 protein. The anti-MCR-1/MCR-2 protein mouse mAbs had good performance in all aspects and were suitable for use as an immunodiagnostic reagent in the preparation of *in vitro* diagnostic kits, which can be made into colloidal gold immunoassay kits, chemiluminescence kits, radioimmunoassay kits, enzyme-linked immunoassay kits, fluorescence immunoassay kits, or microfluidic chips. The kits prepared can be used to detect the MCR-1/MCR-2 proteins. The two antibodies involved in the present solution are especially suitable for making a double-antibody sandwich immuno-colloidal gold test card, where the antibody 2DG5 is used as a coated antibody, and the antibody 1IB10 is used as a gold-labeled antibody. The produced double-antibody sandwich immuno-colloidal gold test card is more sensitive. Similarly, the antibody 2DG5 can also be used as the gold-labeled antibody, and the antibody 1IB10 can be used as the coated antibody.

The present invention is further explained by specific embodiments below. Specifically, the experimental methods where operation steps are not specified are carried out in accordance with the corresponding product specifications. The instruments, reagents, and consumables used in the embodiments can be purchased from commercial companies unless otherwise specified.

### Embodiment 1: Preparation of the anti-MCR-1/MCR-2 protein mouse mAbs

### 1.1 Antigen preparation

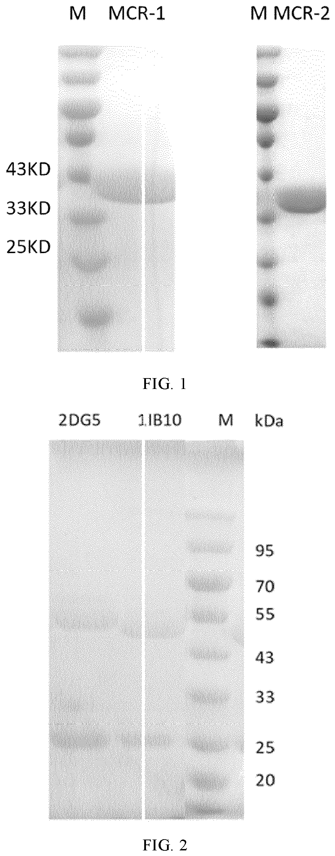

The mcr-1 type gene sequence and the mcr-2 type gene sequence were found and downloaded from National Center for Biotechnology Information (NCBI), and the recombinant plasmids were transformed into *Escherichia coli* (*E. coli*) for induced expression. The method for purification was by using nickel column affinity chromatography, the bacterial body was resuspended in an equilibrium buffer solution with a pH of 7.4, ultrasonic was carried out until the bacterial solution was clarified, and then centrifuged at a high speed. After the resulting supernatant was passed over a membrane, then over a nickel column. The target protein was eluted out, and the molecular weight was consistent with the predicted molecular weight. Sodium dodecyl sulfate-polyacrylamide gel electrophoresis (SDS-PAGE) is shown in FIGS. 1-2, and the target protein was quantified by a bicinchoninic acid (BCA) method and sub-packaged.

### 1.2 Mouse immunity

Female BALB/c mice, aged about six weeks, were immunized with the purified MCR-1 protein for antibody preparation. According to the immunization dose, the mice were divided into two groups, with 5 mice in each group. According to the antigen content, the immunization dose of the first group was 25 µg/mouse, and the immunization dose of the second group was 50 µg/mouse. For the first immunization, an appropriate amount of the MCR-1 protein was diluted to 500 µL by normal saline, the same amount of 500 µL of Freund's complete adjuvant was added to for thorough emulsification, and the mice were immunized by subcutaneous injection at multiple points. Two weeks later, the second immunization was carried out with the same dose and the mice were immunized by intraperitoneal injection, where the Freund's complete adjuvant was replaced with the Freund's incomplete adjuvant. Two weeks later, the mice were immunized again by intraperitoneal injection. Seven days later, blood was collected from the mouse tail, and the enzyme-linked immunosorbent assay (ELISA) was used to measure the mouse serum titer. The specific procedure was as follows: The ELISA plate was subjected to coating overnight at 4°C with 100 µL of 0.2 µg/mL MCR-1 protein per well, and the resulting ELISA plates were shaken for drying and washed 3 times with phosphate buffered saline with tween-20 (PBST). The resulting ELISA plate was blocked at 37°C for 2 h with 200 µL of 5% skimmed milk powder per well. Blood was collected from the mouse tail and centrifuged at 3000 r/min, and serum was collected after centrifugation and doubling diluted in PBS from 1:1000 to 1:512000 for later use. The resulting ELISA plate was shaken for drying and washed 3 times with PBST. The primary antibody diluted in PBS was added in the resulting ELISA plate from 1:1000, with 100 µL per well, and incubated at 37°C for 1 h. The resulting ELISA plate was shaken for drying and washed 3 times with PBST. The goat anti-mouse secondary antibody diluted in PBS at 1:10000 was added in the resulting ELISA plate, with 100 µL per well, and incubated at 37°C for 45 min. The resulting ELISA plate was shaken for drying and washed 5 times with PBST. 3,3',5,5'-tetramethylbenzidine (TMB) was added to the resulting ELISA plate, with 100 µL per well. Color development was performed at 37°C for 10 minutes, the reaction was terminated, and values were read.

### 1.3 Cell fusion

Three days before fusion, the mice were immunized with the same vaccination amount as the previous immunization without adjuvant by intraperitoneal injection. One day before fusion, the feeder layer cells were prepared, and one BALB/c mouse aged 6-8 weeks was taken and killed by cervical dislocation after eyeball exsanguination. The dead BALB/c mouse was sterilized in 75% alcohol for 5 minutes and fixed on a plate, and the abdominal skin was cut aseptically in an ultra-clean table. Ten mL of hypoxanthine-aminopterin-thymidine (HAT) selection medium was absorbed by an aseptic syringe and injected into the abdominal cavity of the dead BALB/c mouse, followed by gently rubbing the abdomen with alcohol cotton balls, withdrawing the medium, and adding the withdrawn medium to 40 mL of HAT medium. The resulting medium was spread into four 96-well cell culture plates with 100 µL per well and incubated in a 5% CO₂ cell incubator at 37°C. One week before fusion, myeloma cells (Sp2/0 cells) were resuscitated, cultured in a PRMI-1640 medium containing 10% fetal bovine serum, and subcultured in a 5% CO₂ incubator at 37°C. The cells in the logarithmic growth stage were collected into a centrifuge tube, cell counting was performed and diluted to 10⁷ cells/mL for later use. The BALB/c mouse immunized for 3 days was taken, and the positive serum was prepared by eyeball exsanguination. The mouse was killed by cervical dislocation and sterilized in 75% alcohol for 5 minutes, and the spleen was removed aseptically on the ultra-clean table. The spleen was washed several times in a sterile plate and the connective tissue was stripped. The resulting spleen was placed on a microporous copper net and fresh RPMI-1640 medium was added. The medium was absorbed by a syringe and injected into an end of the spleen and the spleen cells were blown down. After repeating the operations several times, the remaining spleen was gently ground with the inner plug of the syringe until there was no obvious red tissue mass. The spleen cell suspension from the plate was gently blown and transferred to a 50 mL centrifuge tube and centrifuged at 1000 r/min for 5 minutes to collect the spleen cells and counted for later use. The spleen cells of the immunized mouse and the Sp2/0 cells were mixed at a cell number ratio of 10:1, added into a 50 mL centrifuge tube, and centrifuged at 1000 r/min for 5 minutes. The resulting supernatant was discarded, and the centrifuge tube was gently rubbed in the palm to make the two cells fully mixed. The centrifuge tube was placed in a 100 mL blue-capped bottle containing 37°C hot water. 1 mL of preheated dimethyl sulfoxide/polyethylene glycol (DMSO/PEG) was added into a fusion tube dropwise within 1 minute, slowly at first and then quickly, and the centrifuge tube was gently rotated while adding the preheated DMSO/PEG. Subsequently, the reaction was terminated by immediately adding an antibody-free and blood-free RPMI-1640 medium at an amount of 1 mL for the first minute, 2 mL for the second minute, 3 mL for the third minute, and 4 mL for the fourth minute. The resulting product was placed in a water bath for 5 minutes at 37 °C and centrifuged at 800 r/min for 5 minutes, the resulting supernatant was discarded, and the resulting precipitate was suspended by HAT. The resulting mixture was mixed into 40 mL of an HAT selection medium containing 20% fetal bovine serum that was preheated at 37°C and spread into the 96-well cell culture plates that was filled with feeder layer cells at a dose of 100 µL per well. The culture plates were put into a 5% CO₂ incubator at 37°C for incubation. Seven days later, fresh HAT medium was used to replace half of media in the culture plates. Ten days later, thymidine (HT) medium was used to replace all media in the culture plates. The positive cells in the 96-well plates were subcloned by a limiting dilution method: First, the feeder layer cells were prepared according to the above method, and the hybridoma cells to be cloned were counted. The cells were diluted to 5-8 cells/mL with HT medium and added to the 96-well cell culture plates that were filled with feeder layer cells at a dose of 100 µL per well. Each hybridoma cell line was cloned into one 96-well cell culture plate and cultured in a 5% CO₂ cell incubator at 37°C. After about 5 days, the number of clones in the cell wells was counted and the clones were labeled. At the seventh day, the medium was replaced with a new medium. The detection was performed when 1/3 to 1/2 of the well bottom was covered by the cells. When all cell wells in the 96-well plates were positive by 2-3 times of cloning, an expanded culture could be carried out, followed by fixing and freezing the cell lines. The hybridoma cells that were tested as positive were subjected to the expanded culture and freezing. The specific process is as follows: The hybridoma cells in vigorous growth and good condition were gently blown off the cell bottle with antibody-free and blood-free Dulbecco's modified eagle medium (DMEM) and centrifuged at 1000 r/min for 5 minutes. The resulting supernatant was discarded. A freezing medium (containing 40% RPMI-1640 medium, 50% fetal bovine serum, and 10% DMSO) was added, and the cells were blown out and sub-packaged into freezing tubes. The freezing tubes were placed in a freezing box and placed in a -70°C refrigerator. One day later, the freezing tubes were transferred to liquid nitrogen and recorded.

### 1.4 Preparation of ascites

Female BALB/c mice, aged 10-12 weeks, were intraperitoneally injected with sterile liquid paraffin at a dose of 0.5 mL/mouse. Seven days later, the hybridoma cells cultured to the logarithmic stage were intraperitoneally injected at a dose of 5×10⁶ cells/mouse. The mice were observed every day. About 7-10 days later, when the abdomen of the mice noticeably swelled, the lower abdominal skin was disinfected with 75% alcohol cotton balls, and a 16-gauge needle was used to penetrate the abdominal cavity to collect ascites. After the ascites was regenerated and accumulated, the ascites was collected again. The collected ascites was centrifuged at 3000 r/min for 10 minutes, and the clarified part in the middle was taken, followed by filtering through a filter paper, sub-packaging, and storing at -70°C.

### 1.5 Antibody purification

Ascites was purified using a Protein-G column through the following steps: 2 mL of ascites was taken and centrifuged at 10000 g. The clarified part was taken and added with 2 mL of wash buffer for thorough mixing. The column was drained with 20% ethanol and equilibrated with 8 mL of wash buffer. The sample was passed through the column and settled at a flow rate of 8S/drop. The sample was repeatedly loaded 3 times, and then 15 mL of wash buffer was used to wash the precipitate at a flow rate of 8S/drop. After washing, 10 mL of elution buffer was used for elution. After elution, the resulting mixture was adjusted to a pH of 7.4 with 1 M Tris having a pH of 9, concentrated with a concentrated column, and dialyzed overnight at 4°C in a 50 kDd dialysis bag filled with PBS.

### Embodiment 2: Identification of the anti-MCR-1/MCR-2 protein mouse mAbs

### 2.1 Identification of antibody subclasses

According to the instructions of the SIGMA kit, the subclass of the mAb was identified by an ELISA capture method as follows: The mAb subclass identification reagent was diluted at 1:1000 and added into the enzyme-labeled wells at a dose of 100 µL per well. The resulting enzyme-labeled wells were incubated at 37°C for 1 h, washed 3 times with PBST, and pat for drying. The antibody was diluted at 1:1000 and added into the resulting enzyme-labeled wells at a dose of 100 µL per well. The resulting enzyme-labeled wells were incubated at 37°C for 1 h, washed 3 times with PBST, and pat for drying. The goat anti-mouse immunoglobulin G-horseradish peroxidase (IgG-HRP) was diluted at 1:10000 and added at a dose of 100 µL per well. The resulting enzyme-labeled wells were incubated at room temperature for 30 min. The color development lasted for 10-20 minutes. The subclass type of the mAb was determined the same as that of subclass reagent added to the wells that had the maximum OD₄₅₀ value. The antibody subtype of the antibody 2DG5 and the antibody 1IB10 was immunoglobulin G1 (IgG1).

### 2.2 Antibody titer determination

The antibody titer was determined by an indirect ELISA method after purification through the following steps: The MCR-1 protein and the MCR-2 protein were diluted to 0.2 µg/mL, respectively, added to wells at a dose of 100 µL per well, and a non-coated control was set up. The resulting wells were coated at 4°C overnight, shaken for drying, and washed 3 times with PBST. Then the resulting wells were blocked at 37°C for 2 h with 5% skimmed milk powder at a dose of 200 µL per well, shaken for drying, and washed 3 times with PBST. Antibodies (initial concentration of 1 mg/mL) were subjected to doubling dilution starting from 1:1000 twelve times and each dilution was added at a dose of 100 µL per well. Meanwhile, a non-coated control was set up. The resulting wells were incubated at 37°C for 1 h, shaken for drying, and washed 3 times with PBST. Goat anti-mouse secondary antibody diluted in PBS at 1:10000 was added at a dose of 100 µL per well, incubated at 37°C for 45 minutes, shaken for drying, and washed 5 times with PBST. TMB was added at a dose of 100 µL per well. Color development was performed at 37°C for 10 minutes. The reaction was terminated, and values were read. After purification, the antibody was diluted to 1 mg/mL, the titers of the MCR-1 protein and the MCR-2 protein were detected, respectively, and the titer reached more than 1:1280000.

### 2.3 Identification of purity and molecular weight of antibody

SDS-PAGE was used to identify the molecular weight and purity of the antibody. For gel preparation, a concentration of the separated gel was 12% and a concentration of the concentrated gel was 5% . For sample preparation, 20 µL of sample and 20 µL of buffer were thoroughly mixed and boiled for 3 minutes. Each well was loaded with 20 µL of the sample and a protein pre-staining marker control was set up. Electrophoresis was carried out at 80 V for 30 minutes and 120 V for 2 h. After the electrophoresis, the samples were stained with Coomassie brilliant blue solution. Decolorization was performed by boiling with deionized water for 5 minutes each time and 3 times in total. The molecular weight of the heavy chain of IgG antibody is generally 50-75 kDa, and the molecular weight of the light chain of the IgG antibody is about 25 kDa. The purified mAb was identified by SDS-PAGE. As shown in FIG. 2, and the antibody 2DG5 and the antibody 1IB10 have clear bands at 50-75 kDa and about 25 kDa, respectively.

### Embodiment 3: Genetic validation of the anti-MCR-1/MCR-2 protein mouse mAbs

Ig variable region genes were cloned by a reverse transcription-polymerase chain reaction (RT-PCR) method. Total RNA was extracted, and the single-stranded complementary DNA (cDNA) was synthesized. Total RNA of 2DG5 and 1IB10 produced by hybridoma cell lines was extracted using a Trizol method (kit purchased from Invitrogen) and reversed into cDNA libraries using Moloney murine leukemia virus reverse transcriptase (M-MLV RT) (purchased from Invitrogen).
Upstream primer of the heavy chain backbone region
   P1: 5'SAGGTGMAGCTKCASSARTCWGG3', as shown in SEQ ID NO: 21;
Downstream primer of the heavy chain variable region
   P2: 5'TGGGGSTGTYGTTTTGGCTGMRGAGACRGTGA3', as shown in SEQ ID NO: 22;
Upstream primer of the light chain precursor peptide
   P3: 5'ATGGATTTTCAAGTGCAGATTTTCAG3', as shown in SEQ ID NO: 23;
Downstream primer of the light chain variable region
   P4: 5'GGATACAGTTGGTGCAGCATCAGCCCGTTT3', as shown in SEQ ID NO: 24;

PCR reaction system (50 µL) was prepared as follows:
cDNA: 2 µL; upstream primer (10 µM): 2 µL; downstream primer (10 µM): 2 µL; dNTP mixture: 2 µL; pyrococcus furiosus (pfu) DNA polymerase (5 U/µL): 1 µL; 10×pfu Buffer II: 5 µL; and ddH₂O: supplementation to 50 µL.

Reaction conditions: Predenaturation at 95°C for 5 minutes. Thirty-five cycles of 95°C for 30 s, 58°C for 30 s, and 72°C for 1 minute. Finally, extension at 72°C for 10 minutes.

The heavy chain variable region (VH) fragments and the light chain variable region (VL) fragments were separated and recovered by agarose gel electrophoresis. The recovered VL and VH fragments were ligated with pMD19-T (sKPCle) carrier (Takara Company), respectively, and the ligation system was as follows:
70 ng of the VL PCR product or 70 ng of the VH PCR product, respectively,1 µL of the pMD19-T (sKPCle) carrier, 5 µL of Solution I ligation reaction solution, and ddH₂O being supplemented to 10 µL. The ligation was performed overnight at 4°C.

The ligated product was transformed into *E.coli* DH5α competent bacteria and cultured overnight at 37°C. Subsequently, a single colony was selected and shaken at 37°C for 2 h. The bacterial liquid was identified by PCR, and the cDNA of the corresponding antibody was used as the positive control. The reaction system (25 µL) was prepared as follows:
Bacteria liquid: 1 µL; upstream primer (10 µM): 1 µL; downstream primer (10 µM): 1 µL; dNTP mixture (each of the dNTPs is 2.5 Mm): 2 µL; Taq DNA polymerase (5 U/µL): 0.5 µL; 10×Taq Buffer (Mg²⁺ plus): 2.5 µL; and water supplement to 25 µL. The reaction conditions were the same as previous.

The PCR-positive clones were selected for expanded culture, and the positive clone plasmids were extracted by plasmid extraction kit (Takara Company) for sequencing. At least five cloned samples were sequenced for each chain of each antibody until at least three samples have the same sequencing results. The sequences of the heavy chain variable region and the light chain variable region of antibodies 2DG5 and 1IB10 were successfully cloned, which were consistent with the characteristics of typical antibody variable region sequences.

### Embodiment 4: Preparation of MCR-1/MCR-2 protein test card by the colloidal gold method

The double-antibody sandwich immuno-colloidal gold test card was prepared as follows:
Step 1, A 0.1 M K₂CO₃ solution was added to a colloidal gold solution while stirring. After adjusting the pH value, the anti-MCR-1 protein mAb 1IB10 was added. After stirring, a 10% bovine serum albumin solution and 2% PEG 20000 were added. After stirring, the resulting mixture was centrifuged at a low speed to collect a supernatant and then the resulting supernatant was centrifuged at a high speed to collect a precipitate. The colloidal gold resuspension was used to reach the predetermined volume to form a gold-labeled antibody.
Step 2, The gold-labeled antibody was sprayed on a glass cellulose film and dried to make a gold-labeled pad.
Step 3, The anti-MCR-1/MCR-1 protein mouse mAb 2DG5 was added with a 1% thiomersal sodium solution and mixed well to form a test line coating solution. Then PBS and a 1% thiomersal sodium solution were added to the goat anti-mouse IgG and mixed well to form a quality control line coating solution. The quality control line coating solution and the test line coating solution were drawn on a nitrate cellulose film and dried to obtain a coated film.
Step 4, The coated film was attached to the bottom plate, the gold-labeled pad and the absorbent paper were attached to the coated film for lamination, and the resulting plate was cut to obtain the MCR-1/MCR-2 protein test card by the colloidal gold method.

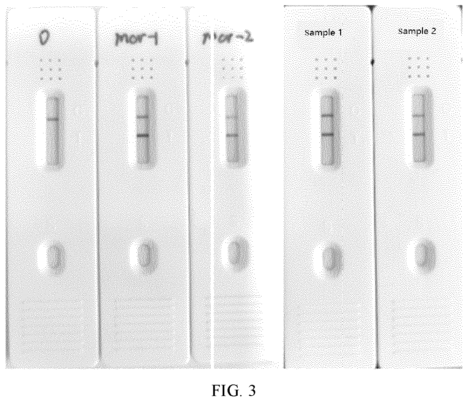

The MCR-1/MCR-2 protein test card prepared by the colloidal gold method above was taken and spotted with a blank sample, an MCR-1antigen, an MCR-2 antigen, a positive sample 1 containing MCR-1 protein, and a positive sample 2 containing MCR-2 protein, respectively. The results, as shown in FIG. 3 from left to right, are the blank sample, the MCR-1antigen, the MCR-2 antigen, the positive sample 1 containing MCR-1 protein, and the positive sample 2 containing MCR-2 protein. It can be seen that the detection result of the blank sample is negative and the results of the MCR-1antigen, the MCR-2 antigen, the positive sample 1 containing MCR-1 protein, and the positive sample 2 containing MCR-2 protein are positive. It is proved that the MCR-1/MCR-2 protein test card prepared by the colloidal gold method in the present solution can detect the MCR-1/MCR-2 protein and the corresponding positive samples.

## Claims

1. An anti-MCR-1/MCR-2 protein mouse mAb, **characterized in that** the anti-MCR-1/ MCR-2 protein mouse mAb is: an antibody 2DG5 compr sing a light chain variable region and a heavy chain variable region; wherein the light chain variable region comprises CDRL1 as shown in SEQ ID NO: 1, CDRL2 as shown in SEQ ID NO: 2, and CDRL3 as shown in SEQ ID NO: 3; the heavy chain variable region comprises CDRH1 as shown in SEQ ID NO: 4, CDRH2 as shown in SEQ ID NO: 5, and CDRH3 as shown in SEQ ID NO: 6; an amino acid sequence of the light chain variable region of the antibody 2DG5 is shown in SEQ ID NO: 7, and an amino acid sequence of the heavy chain variable region of the antibody 2DG5 is shown in SEQ ID NO: 9
or
an antibody 1IB10, comprising a light chain variable region and a heavy chain variable region, wherein the light chain variable region comprises CDRL1 as shown in SEQ ID NO: 11, CDRL2 as shown in SEQ ID NO: 12, and CDRL3 as shown in SEQ ID NO: 13; the heavy chain variable region comprises CDRH1 as shown in SEQ ID NO: 14, CDRH2 as shown in SEQ ID NO: 15, and CDRH3 as shown in SEQ ID NO: 16; an amino acid sequence of the light chain variable region of the antibody 1IB10 is shown in SEQ ID NO: 17, and an amino acid sequence of the heavy chain variable region is shown in SEQ ID NO: 19.

2. The anti-MCR-1/MCR-2 protein mouse mAb according to claim 1, **characterized in that** the antibody 2DG5 is produced by an anti-MCR-1/MCR-2 protein mouse hybridoma cell with an accession number of CGMCC No. 23034; and
the antibody 1IB10 is produced by an anti-MCR-1/MCR-2 protein mouse hybridoma cell with an accession number of CGMCC No. 23032.

3. A nucleic acid molecule, comprising: a nucleotide encoding the anti-MCR-1/MCR-2 protein mouse mAb according to claim 1.

4. The nucleic acid molecule according to claim 3, **characterized in that** a nucleotide sequence of the nucleic acid molecule encoding the light chain variable region of the antibody 2DG5 is shown in SEQ ID NO: 8, and a nucleotide sequence of the nucleic acid molecule encoding the heavy chain variable region of the antibody 2DG5 is shown in SEQ ID NO: 10;
or,
a nucleotide sequence of the nucleic acid molecule encoding the light chain variable region of the antibody 1IB10 is shown in SEQ ID NO: 18, and a nucleotide sequence of the nucleic acid molecule encoding the heavy chain variable region of the antibody 1IB10 is shown in SEQ ID NO: 20.

5. Use of the anti-MCR-1/MCR-2 protein mouse mAb according to any one of claims 1 to 2 in a preparation of reagents for *in vitro* detection of an MCR-1 or MCR-2 protein.

6. The use of the anti-MCR-1/MCR-2 protein mouse mAb in the preparation of the reagents for detection of an MCR-1 or MCR-2 protein according to claim 5, **characterized in that** the anti-MCR-1/MCR-2 protein mouse mAb is used to prepare an *in vitro* diagnostic kit or a microfluidic chip; the *in vitro* diagnostic kit is a colloidal gold immunoassay kit, a chemiluminescence kit, a radioimmunoassay kit, an enzyme-linked immunoassay kit, or a fluorescence immunoassay kit.

7. The use of the anti-MCR-1/MCR-2 protein mouse mAb in the preparation of reagents for detection of an MCR-1 or MCR-2 protein according to claim 5, **characterized in that** in a preparation of a double-antibody sandwich immuno-colloidal gold test card, the antibody 2DG5 is used as a coated antibody, and the antibody 1IB10 is used as a gold-labeled antibody;
or the antibody 1IB10 is used as the coated antibody, and the antibody 2DG5 is used as the gold-labeled antibody.

## Patentansprüche

1. Ein Anti-MCR-1/MCR-2-Protein-Maus-mAb, **dadurch gekennzeichnet, dass** das Anti-MCR-1/MCR-2-Protein-Maus-mAb ist: ein Antikörper 2DG5, der eine leichte Ketten-Variable-Region und eine schwere Ketten-Variable-Region umfasst; wobei
die leichte Ketten-Variable-Region CDRL1, gezeigt in SEQ ID NO: 1, CDRL2, gezeigt in SEQ ID NO: 2, und CDRL3, gezeigt in SEQ ID NO: 3 umfasst; die schwere Ketten-Variable-Region umfasst CDRH1, gezeigt in SEQ ID NO: 4, CDRH2, gezeigt in SEQ ID NO: 5, und CDRH3, gezeigt in SEQ ID NO: 6; eine Aminosäuresequenz der leichten Ketten-Variable-Region des Antikörpers 2DG5 ist in SEQ ID NO: 7 gezeigt, und eine Aminosäuresequenz der schweren Ketten-Variable-Region des Antikörpers 2DG5 ist in SEQ ID NO: 9 gezeigt,
oder
ein Antikörper 1IB10, der eine leichte Ketten-Variable-Region und eine schwere Ketten-Variable-Region umfasst, wobei die leichte Ketten-Variable-Region CDRL1, gezeigt in SEQ ID NO: 11, CDRL2, gezeigt in SEQ ID NO: 12, und CDRL3, gezeigt in SEQ ID NO: 13 umfasst; die schwere Ketten-Variable-Region umfasst CDRH1, gezeigt in SEQ ID NO: 14, CDRH2, gezeigt in SEQ ID NO: 15, und CDRH3, gezeigt in SEQ ID NO: 16; eine Aminosäuresequenz der leichten Ketten-Variable-Region des Antikörpers 1IB10 ist in SEQ ID NO: 17 gezeigt, und eine Aminosäuresequenz der schweren Ketten-Variable-Region ist in SEQ ID NO: 19 gezeigt.

2. Anti-MCR-1/MCR-2-Protein-Maus-mAb nach Anspruch 1, **dadurch gekennzeichnet, dass** der Antikörper 2DG5 durch eine Anti-MCR-1/MCR-2-Protein-Maus-Hybridomzelle mit der Hinterlegungsnummer CGMCC No. 23034 produziert wird; und
Antikörper 1IB10 durch eine Anti-MCR-1/MCR-2-Protein-Maus-Hybridomzelle mit der Hinterlegungsnummer CGMCC No. 23032 produziert wird.

3. Ein Nukleinsäuremolekül, umfassend: ein Nukleotid, das das Anti-MCR-1/MCR-2-Protein-Maus-mAb nach Anspruch 1 kodiert.

4. Nukleinsäuremolekül nach Anspruch 3, **dadurch gekennzeichnet, dass** eine Nukleotidsequenz des Nukleinsäuremoleküls, das die leichte Ketten-Variable-Region des Antikörpers 2DG5 kodiert, die in SEQ ID NO: 8 gezeigt ist, und eine Nukleotidsequenz des Nukleinsäuremoleküls, das die schwere Ketten-Variable-Region des Antikörpers 2DG5 kodiert, in SEQ ID NO: 10 gezeigt;
oder,
eine Nukleotidsequenz des Nukleinsäuremoleküls, das die leichte Ketten-Variable-Region des Antikörpers 1IB10 kodiert, die in SEQ ID NO: 18 gezeigt ist, und eine Nukleotidsequenz des Nukleinsäuremoleküls, das die schwere Ketten-Variable-Region des Antikörpers 1IB10 kodiert, in SEQ ID NO: 20.

5. Verwendung des Anti-MCR-1/MCR-2-Protein-Maus-mAb nach einem der Ansprüche 1 bis 2 bei der Herstellung von Reagenzien zur *in-vitro*-Detektion eines MCR-1- oder MCR-2-Proteins.

6. Verwendung des Anti-MCR-1/MCR-2-Protein-Maus-mAb bei der Herstellung der Reagenzien zur Detektion eines MCR-1- oder MCR-2-Proteins nach Anspruch 5, **dadurch gekennzeichnet, dass** das Anti-MCR-1/MCR-2-Protein-Maus-mAb zur Herstellung eines in-vitro-Diagnostikumsatzes oder eines mikrofluidischen Chips verwendet wird; das in-vitro-Diagnostikumsatz ist ein kolloidaler Gold-Immunoassay-Satz, ein Chemilumineszenzsatz, ein Radioimmunoassay-Satz, ein Enzyme-linked-Immunoassay-Satz oder ein Fluoreszenz-Immunoassay-Satz.

7. Verwendung des Anti-MCR-1/MCR-2-Protein-Maus-mAb bei der Herstellung von Reagenzien zur Detektion eines MCR-1- oder MCR-2-Proteins nach Anspruch 5, **dadurch gekennzeichnet, dass** bei der Herstellung einer Doppelantikörper-Sandwich-Immuno-Kolloidgold-Testkarte der Antikörper 2DG5 als Beschichtungsantikörper verwendet wird und der Antikörper 1IB10 als goldmarkierter Antikörper verwendet wird;
oder der Antikörper 1IB10 als Beschichtungsantikörper verwendet wird und der Antikörper 2DG5 als goldmarkierter Antikörper verwendet wird.

## Revendications

1. Anticorps monoclonal protéique anti-MCR-1/MCR-2 de souris, **caractérisé en ce que** l'anticorps monoclonal protéique anti-MCR-1/MCR-2 de souris est : un anticorps 2DG5 comprenant une région variable de chaîne légère et une région variable de chaîne lourde ; dans lequel
la région variable de chaîne légère comprend CDRL1 telle que représentée dans SEQ ID NO: 1, CDRL2 telle que représentée dans SEQ ID NO: 2, et CDRL3 telle que représentée dans SEQ ID NO: 3 ; la région variable de chaîne lourde comprend CDRH1 telle que représentée dans SEQ ID NO: 4, CDRH2 telle que représentée dans SEQ ID NO: 5, et CDRH3 telle que représentée dans SEQ ID NO: 6 ; une séquence d'acides aminés de la région variable de chaîne légère de l'anticorps 2DG5 est représentée dans SEQ ID NO: 7, et une séquence d'acides aminés de la région variable de chaîne lourde de l'anticorps 2DG5 est représentée dans SEQ ID NO: 9,
ou
un anticorps 1IB10, comprenant une région variable de chaîne légère et une région variable de chaîne lourde, dans lequel la région variable de chaîne légère comprend CDRL1 telle que représentée dans SEQ ID NO: 11, CDRL2 telle que représentée dans SEQ ID NO: 12, et CDRL3 telle que représentée dans SEQ ID NO: 13 ; la région variable de chaîne lourde comprend CDRH1 telle que représentée dans SEQ ID NO: 14, CDRH2 telle que représentée dans SEQ ID NO: 15, et CDRH3 telle que représentée dans SEQ ID NO: 16 ; une séquence d'acides aminés de la région variable de chaîne légère de l'anticorps 1IB10 est représentée dans SEQ ID NO: 17, et une séquence d'acides aminés de la région variable de chaîne lourde est représentée dans SEQ ID NO: 19.

2. Anticorps monoclonal protéique anti-MCR-1/MCR-2 de souris selon la revendication 1, **caractérisé en ce que** l'anticorps 2DG5 est produit par une cellule d'hybridome protéique anti-MCR-1/MCR-2 de souris avec un numéro d'accès CGMCC n° 23034 ; et
l'anticorps 1IB10 est produit par une cellule d'hybridome protéique anti-MCR-1/MCR-2 de souris avec un numéro d'accès CGMCC n° 23032.

3. Molécule d'acide nucléique, comprenant : un nucléotide codant pour l'anticorps monoclonal protéique anti-MCR-1/MCR-2 de souris selon la revendication 1.

4. Molécule d'acide nucléique selon la revendication 3, **caractérisée en ce qu'**une séquence nucléotidique de la molécule d'acide nucléique codant pour la région variable de chaîne légère de l'anticorps 2DG5 est représentée dans SEQ ID NO: 8, et une séquence nucléotidique de la molécule d'acide nucléique codant pour la région variable de chaîne lourde de l'anticorps 2DG5 est représentée dans SEQ ID NO: 10 ;
ou,
une séquence nucléotidique de la molécule d'acide nucléique codant pour la région variable de chaîne légère de l'anticorps 1IB10 est représentée dans SEQ ID NO: 18, et une séquence nucléotidique de la molécule d'acide nucléique codant pour la région variable de chaîne lourde de l'anticorps 1IB10 est représentée dans SEQ ID NO: 20.

5. Utilisation de l'anticorps monoclonal protéique anti-MCR-1/MCR-2 de souris selon l'une quelconque des revendications 1 à 2 dans une préparation de réactifs pour la détection *in vitro* d'une protéine MCR-1 ou MCR-2.

6. Utilisation de l'anticorps monoclonal protéique anti-MCR-1/MCR-2 de souris dans la préparation de réactifs pour la détection d'une protéine MCR-1 ou MCR-2 selon la revendication 5, **caractérisée en ce que** l'anticorps monoclonal protéique anti-MCR-1/MCR-2 de souris est utilisé pour préparer un kit de diagnostic *in vitro* ou une puce micro fluidique ; le kit de diagnostic *in vitro* est un kit de dosage immunologique d'or colloïdal, un kit de chimioluminescence, un kit de dosage radioimmunologique, un kit de dosage immunologique lié à une enzyme, ou un kit de dosage immunologique par fluorescence.

7. Utilisation de l'anticorps monoclonal protéique anti-MCR-1/MCR-2 de souris dans la préparation de réactifs pour la détection d'une protéine MCR-1 ou MCR-2 selon la revendication 5, **caractérisée en ce que**, dans une préparation d'une carte de test d'or immuno-colloïdal sandwich à double anticorps, l'anticorps 2DG5 est utilisé comme anticorps revêtu, et l'anticorps 1IB10 est utilisé comme anticorps marqué à l'or ;
ou l'anticorps 1IB10 est utilisé comme anticorps revêtu, et l'anticorps 2DG5 est utilisé comme anticorps marqué à l'or.
